# EUROPEAN PATENT APPLICATION

(11) **EP 4 502 935 A1**
(43) Date of publication of application: **05.02.2025**
(21) Application number: 24192240.0
(22) Date of filing: 01.08.2024
(51) Int. Cl.: G06T 7/00, G06T 11/00

(54) **MEDICAL IMAGE SYNTHESIS**

(30) Priority: 04.08.2023 US 202318365278
(71) Applicant: Siemens Medical Solutions USA, Inc., Malvern, PA 19355 (US)
(72) Inventor: ZHAO, Gengyan, Plainsboro, NJ, 08536 (US); GIBSON, Eli, Plainsboro, NJ, 08536 (US); MAILHE, Boris, Plainsboro, NJ, 08536 (US); YOO, Youngjin, Princeton, NJ, 08540 (US); GEORGESCU, Bogdan, Princeton, NJ, 08540 (US); COMANICIU, Dorin, Princeton, NJ, 08540 (US)
(74) Representative: Horn Kleimann Waitzhofer Schmid-Dreyer Patent- und Rechtsanwälte PartG mbB

(57) **Abstract**

A system (100) for synthesizing medical images including synthesizing medical abnormalities has multiple diffusion model based denoising stages (110, 150). At a first denoising stage (110), a machine-learned network denoises (210) a first noise input (112) to obtain an abnormality spatial mask (140) detailing positional and structural characteristics of the synthesized medical abnormality. At a second denoising stage (150), a machine-learned network denoises (220) a second noise input (152) based on the abnormality spatial mask (140) and a pre-abnormality image (160) to obtain a synthesized medical image (170) that corresponds to the pre-abnormality image (160) with the synthesized medical abnormality inserted consistent with the abnormality spatial mask (140).

## Description

### STATEMENT ON GOVERNMENT SUPPORT

This invention is made with government support under R01CA262182 awarded by the National Institutes of Health. The government has certain rights in the invention.

### TECHNICAL FIELD

This disclosure relates to medical image synthesis.

### BACKGROUND

Detection of various types of tumors and/or other medical abnormalities is important due to the morbidity and mortality associated with various conditions. Rapid progress has occurred in the field of deep learning (DL), and DL based models are increasingly used in medical abnormality classification, segmentation and detection to facilitate diagnosis and treatment. However, in many cases, the availability of suitable training data, e.g., images with abnormalities, may be limited. For example, older images may lack the resolution and/or detail needed for training. For example, various images showing known types of abnormalities may be absent among available images. For example, privacy laws and regulations may bar use of images without patient permission. Thus, there is demand for sources of quality medical images that may be used freely and that comprehensively cover a spectrum of conditions.

### BRIEF DESCRIPTION OF THE DRAWINGS

Figure 1 shows an illustrative example image synthesis system.
Figure 2 shows example image synthesis logic.
Figure 3 shows an example image synthesis computation environment.
Figure 4 shows an illustrative example image synthesis system.
Figure 5 shows illustrative example training descriptor generation logic.

### DETAILED DESCRIPTION

In various contexts, a set of medical images that contain at least some medical images with medical abnormalities, such as, tumors, lung nodules, lesions, or other medical abnormalities, may be used in various training scenarios. For example, the set of medical images may be used to train medical professionals. In some scenarios, a set of medical images may be used to train a machine learning (ML) system, e.g., for classification, diagnosis, identification, segmentation, and/or various other ML operations. Nevertheless in some scenarios, the availability of images with abnormalities may be limited. In some cases, available images may not be of the needed type for the training application. For example, some images that may fill a deficiency within the training set may be available only as computerized tomography (CT) scans for a training application using magnetic resonance imaging (MRI) images, or vice versa. In some cases, the dimensionality of available images may not match that of the application. For example, two-dimensional images may be available but three-dimensional images may be required. In some cases, the resolution and/or clarity of available images may be insufficient (or otherwise not match the requirements of the application).

For sets requiring images with abnormalities, the problems discussed above may be more acute. Abnormality images, due to their nature, may be more rare than similar non- and/or pre- abnormality images. In some cases, this rarity may occur because populations with particular abnormalities may be smaller than populations lacking the particular abnormalities. Thus, obtaining consistent quality images that cover a variety of positions, structures, sizes, heterogeneity levels, progression levels, and typologies of a particular abnormalities may be difficult. This may inhibit training (e.g., both ML and medical professional training).

Synthetic images may be used to fill deficiencies within an image set. Deficiencies in an image set may occur when the image set has an insufficient number of medical images with abnormalities with selected characteristics. For example, the image set may deviate from a known distribution of characteristics for an abnormality due to having too few medical images of abnormalities with particular characteristics. For example, the image set may lack medical images of abnormalities with the particular characteristics altogether. For example, the image set may have a below threshold amount of medical images of abnormalities with the particular characteristics. In some cases, the threshold used to define a deficiency may correspond to ML training guidelines, statistically derived thresholds, or other thresholds that may be indicative training quality or accuracy.

Conventional systems for ML-based synthetic image generation, may produce images of limited quality and resolution. Conversely, diffusion model image generation based on noise removal may produce images that have comparatively increased resolution. However, diffusion model image generation may produce images without ground-truth segmentation of the abnormality within the synthesized medical image. Thus, medical experts are relied on to review and classify/segment/review the synthesized medical images. Additionally or alternatively, diffusion model image generation (due to the random nature of denoising) produces images that lack control inputs. Thus, creating and or supplementing medical image sets with images of abnormalities with particular characteristics may rely on stochastic generation, which may be inefficient because the system cannot be directed to generate the medical images with the needed characteristics. Instead many images are generated with random characteristics until the desired subset is produced.

The techniques and architectures herein proceed contrary to the conventional wisdom by using a diffusion model based machine-learned denoising system that implements image synthesis with multiple denoising stages. At a first stage, a diffusion model based denoising is performed to generate an abnormality spatial mask that details and segments various volumetric, structural, and typological characteristics of the abnormality. At a second stage, second diffusion model based denoising is performed using the abnormality spatial mask and a pre-abnormality medical image. The second stage inserts the abnormality into the pre-abnormality medical image consistent with the abnormality spatial mask. Thus, the resultant output includes a ground-truth abnormality spatial mask that details and segments various volumetric, structural, and typological characteristics of the synthesized abnormality within the synthesized medical image. The abnormality spatial mask obviates expert analysis of the synthesized medical image to perform identification, structural analysis, segmentation, or other analysis. Accordingly, the output of the multiple-stage diffusion model based denoising is ready for training use without necessarily relying on additional expert analysis. As used herein, "denoising" operations mentioned below may refer to diffusion model based denoising, which is part of the diffusion model.

Additionally or alternatively, the multiple-stage denoising may be used to generate medical images of abnormalities with selected characteristics. At the first stage, in some implementations, a descriptor for one or more selected characteristics of the abnormality may be provided. For example, the descriptor may include one or more spheres (or other objects) indicating one or more size, volume, and/or structural characteristics for the abnormality. For example, the descriptor may include a vector indicating one or more characteristics (structural, clinical, or other observable characteristics) of the abnormality based on definitions for the various entries within the vector. In some cases, image data and/or natural language descriptions may be transformed into descriptors using one or more ML systems. Based on the descriptor, the first denoising stage may generate an abnormality spatial mask with the selected characteristics specified in the descriptor. Consequently, the abnormality depicted in the synthesized medical image may also include the selected characteristics. Thus, in some implementations, the multiple-stage denoising machine-learned system described herein may be used to generate synthesized medical images with selected characteristics. Thus, the multiple-stage structure of the denoising machine-learned system may solve the technical problem of uncontrolled / stochastic medical image generation by implementing the technical feature of parameterized descriptor input for directed abnormality characteristic selection at the denoising stage that generates the abnormality spatial mask.

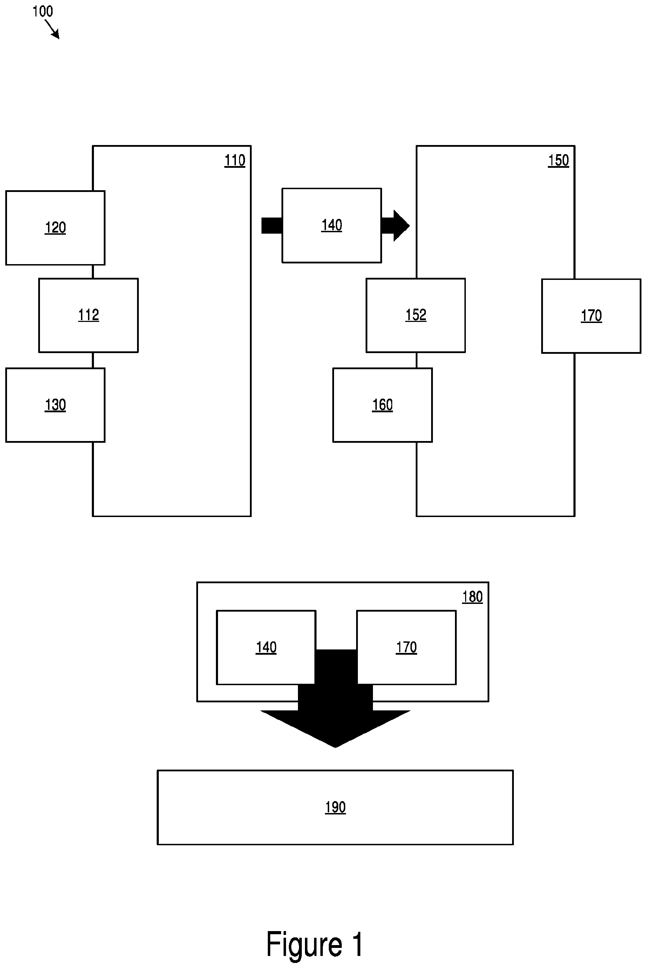

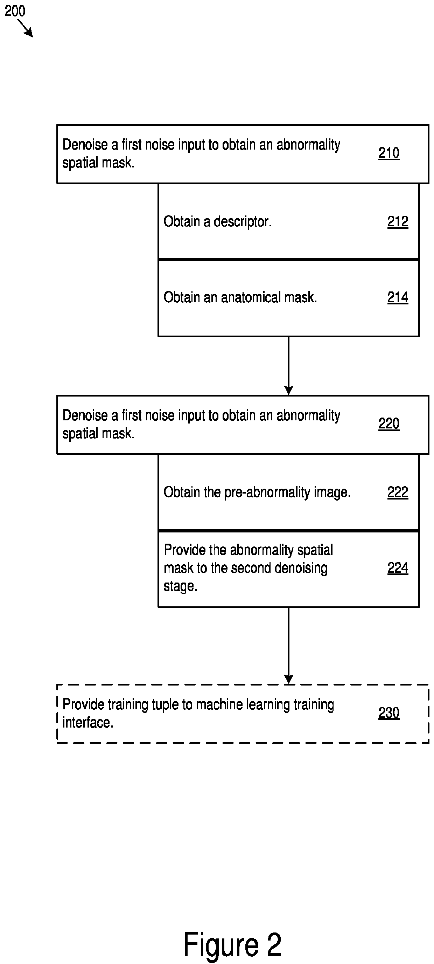

Figure 1 shows an example image synthesis system (ISS) 100. The example ISS 100 includes multiple denoising stages 110, 150. Referring also to Figure 2, example image synthesis logic (ISL) 200 is shown. The ISL 200, which may be implemented on circuitry, may govern the operation of the ISS 100.

In various implementations, the denoising stages may include diffusion model neural networks. The diffusion model neural networks may be trained to remove noise from a noise image. When trained to remove noise to generate a specific type of images, e.g., medical images such as MRI images, CT scans, or other medical images, a diffusion model network may generate a synthesized image of that type from a pure noise input. In various implementations, an iterative denoising process may be used. For example, for a given denoising stage, the ISL 200 may cause the denoising stages to iterative repeat the denoising process to successively remove a selected noise level from the image to progress towards a fully denoised image. In some implementations, a specific neural network for each iteration may be used. In other words, the neural network for each iteration may be specifically trained to remove noise from an image with a specific noise level to produce an image at a specific output noise level (e.g., that may correspond to the input noise level for the next neural network in the iterative chain).

In various implementations, convolutional neural networks may be trained to perform the diffusion model denoising.

The first denoising stage 110 may perform denoising on a first noise input 112 (210). The first noise input may occupy a defined multidimensional space, such as a three-dimensional (3D) volume or a two-dimensional (2D) plane. The defined multidimensional space may include the space occupied by the resultant synthesized medical image. Accordingly, the dimensionality and dimensions of the noise input (e.g., at each of the multiple denoising stages) may match the dimensionality and dimensions of the output synthesized medical image, which may, for example, be a 3D or 2D image. Thus, the output corresponds to a noise-removed input.

In various implementations, the first denoising stage 110 may obtain a descriptor 120 as an input (212). As discussed above, the descriptor may detail selected characteristics of the synthesized abnormality to guide the medical image synthesis to outputs with those selected characteristics. The format of the descriptor may be specific to the denoising system.

For example, the descriptor may be formatted as one or more spheres (or other objects) indicating one or more size, volume, and/or structural characteristics for the abnormality and/or affected tissue of the anatomy surrounding the tissue. In some implementations, the spheres may be concentric. The center of the concentric spheres may indicate a position for a center-of-mass of the abnormality. The volume of each of the one or more spheres may correspond to the volume of a particular segment of the abnormality. For example for a tumor type abnormality, a first sphere may indicate a core density for a tumor, and a second sphere may indicate a density of another peripheral region of the tumor, a third sphere may indicate another volume characteristic of the tumor and/or a volume of non-tumor tissue affected by the insertion of the tumor. Various spheres may be included. In some cases, the spheres may further indicate the total volume of the abnormality and/or the affected tissue region. For example, in a concentric configuration, rather than the volume of each sphere indicating a volume for a segment, the shell formed by a given sphere to the next innermost sphere defines the volume for the segment. Thus, the volume of the innermost sphere defines a first segment volume, then each successive sphere going outward defines a segment volume based on the differential between the volume of that particular sphere and the volume of the sphere just a size smaller. Therefore, the volume of the outermost sphere defines the total volume of the abnormality and/or the affected tissue region.

For example, the descriptor 120 may include a vector indicating one or more characteristics of the abnormality based on definitions for the various entries within the vector. In some cases, image data and/or natural language descriptions may be transformed into descriptors using one or more ML systems. Based on the descriptor, the first denoising stage may generate an abnormality spatial mask 140 with the selected characteristics of the descriptor.

In various implementations, the descriptor 120 may be generated using a large language model (LLM) analysis, a keyword analysis, a natural language processing (NLP) operation, or other language analysis of a clinical description of selected characteristics to generate a descriptor 120 for input to the first denoising stage 110. For example, a LLM analysis may be used to select sphere sizes and position based on clinical notes. Similarly, a vector-type descriptor may be generated using such a language analysis. In an example, language processing may be used to create an interface in which an operator may request medical images with abnormalities with specific characteristics.

In various implementations, operators may compile descriptors directly. For example, an operator may draw spheres and/or generate vectors through direct vector entry input.

The vector and/or transformed text descriptors may be encoded for application to the machine-learned network. In some cases, a trained auto-encoded ML network may be used to encode the vector and/or transformed text descriptors.

In some implementations, ranges for characteristics and/or a defined distribution of abnormality characteristics may be defined. Using the ranges and/or defined distribution, constrained random and/or pseudorandom variable generation may be used to generate descriptor inputs.

In various implementations, the particular way the descriptor is used by the first denoising stage may be controlled via the training of the one or more neural networks on which the first denoising stage is implemented.

In some implementations using multiple-layer neural networks (e.g., deep neural networks), the descriptor 120 may be applied (e.g., in whole or in part) at various layers of the neural network. Thus, the one or more neural networks used by the denoising stages may include inputs at various layers (including hidden layers) within the neural networks. For multiple-iteration denoising stages, the denoising stage may have one or more layer inputs for each of the iterations. The configuration of layer inputs may be consistent throughout iterations for a stage or may change from iteration-to-iteration. For example, the layer structure of the neural networks for the various iterations may be allowed to differ, e.g., to allow for specialization to the particular noise level removal being performed be each of the neural networks.

In some cases, to train the machine-learned networks to perform the denoising, a ground truth image may have set step levels of noise added. Each of the step levels may be used as a training tuple, with the images with one step less noise added being the ground truth for the image with one step more noise added.

In various implementations, the first denoising stage 110 may obtain an anatomical mask 130 as an input (214). The anatomical mask 130 may detail the spatial layout of the anatomical background in the multidimensional space defined in the first noise sample. The anatomy may be used by the first denoising stage 110 to determine positioning and shape of the abnormality. For example, the anatomical mask 130 may include anatomical boundaries. The first denoising stage may shape and position the abnormality to handle the anatomical boundaries. For example, some rigid boundaries, such as a skull in the case of the brain tumor type abnormality: may disallow straddling (e.g., the tumor may not be on be on both sides), may disallow crossing (e.g., the tumor may not be outside the skull), and/or may disallow deformation (e.g., the tumor may not deform the skull). For flexible boundaries, straddling and/or crossing may be disallowed, and deformation of the boundary may be permitted. Other boundaries may allow some selection of crossing, straddling, and/or deformation while disallowing the complementary selection.

In various implementations, the particular way the anatomical mask 130 is used by the first denoising stage may be controlled via the training of the one or more neural networks on which the first denoising stage is implemented.

The second denoising stage 150 may denoise a second noise 152 input using a pre-abnormality image 160 and the abnormality spatial mask 140 as inputs to produce a synthesized medical image 170 (220). Thus for the second denoising stage, the ISL 200 may obtain the pre-abnormality image 160 (222) and provide the abnormality spatial mask to the second denoising stage (224).

The pre-abnormality image 160 may include a medical image, such as an MRI image or a CT scan, of the particular portion of the body prior to insertion of the abnormality. For example, the pre-abnormality image 160 may include a non-abnormality image of a patient with no known conditions. For example, the pre-abnormality image 160 may include a patient with a known condition that may be related or unrelated to abnormality being inserted. The dimensionality and size of the pre-abnormality image 160 may correspond to that of the defined multidimensional space of the first noise input (and the second noise input). Thus, the position and structure of the abnormality spatial mask 140 may be translated to a position and structure within the pre-abnormality image 160.

The pre-abnormality image 160 may be the source used to generate the anatomical mask 130. Thus, the anatomical mask 130 may correspond spatially to the anatomy viewable in within the pre-abnormality image 160.

As discussed above, the denoising process by the second denoising stage may be iterative in some implementations. Thus, the successive iterations may remove a specific level of noise from the second noise input.

The synthesized medical image 170 may correspond to the pre-abnormality image 160 with an abnormality consistent with the abnormality spatial mask 140 inserted. Accordingly, the positioning, structure, and segmentation of the inserted abnormality may be consistent with that in the abnormality spatial mask 140. The synthesized medical image 170 may include regions which are changed relative to the pre-abnormality image 160 to accommodate the abnormality. For example, flexible anatomical boundaries may be shifted to avoid disallowed straddling, tissue may be compressed or expanded to account for the insertion of the abnormality, portions of the pre-abnormality image 160 may be replaced with abnormality structure, and/or other changes may occur. Thus, the resultant synthesized medical image 170 may be similar to and/or indistinguishable from a medical image obtained using an actual scan of a patient having that particular abnormality. In other words, the integration of the pre-abnormality image 160 and the abnormality spatial mask 140 may be more than a layering of the abnormality spatial mask 140 one top of the pre-abnormality image 160.

The second denoising stage may also use descriptor input. For example, a bounding box within the multidimensional space may indicate the regions of pre-abnormality image that may be affected by the insertion of the abnormality. A language or vector descriptor may indicate similar image characteristics. Moreover, descriptors may indicate specific tissue effects and medical conditions that may be present proximate to the abnormality, e.g. mass effects, level of infarction, or other descriptions.

In some implementations used for generation of ML training data, the synthesized medical image 170 and abnormality spatial mask 140 may be provided to a machine learning training interface 190 (e.g., for training other machine learning systems) as a training tuple 180 (230). The training tuple 180 may define the synthesized medical image 170 as a training input and the abnormality spatial mask 140 as a ground truth output. Thus, the training tuple provides a medical image input paired with a segmented/identified abnormality output.

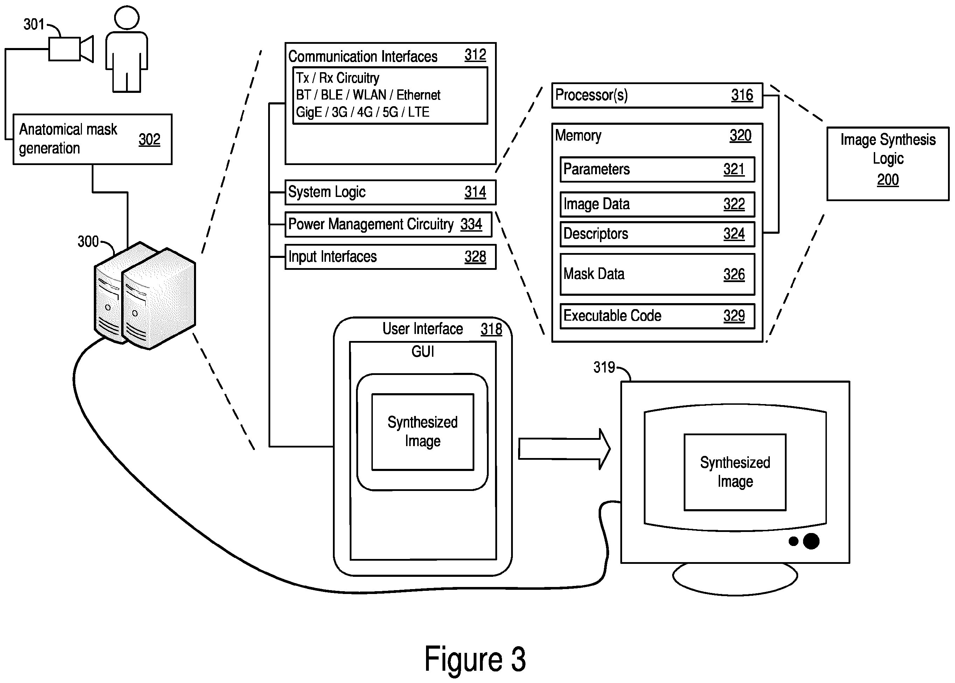

Figure 3 shows an example synthesis computation environment (SCE) 300, which, for example, may operate as synthesis circuitry. The SCE 300 may include system logic 314 to support implementation of the example ISL 200. The system logic 314 may include processors 316, memory 320, and/or other circuitry, which may be used to implement the multiple denoising stages, provide inputs to the stages, pass information between the stages, iterate denoising operations, execute machine-learned networks, and/or perform other image synthesis operations.

In some implementations, the SCE 300 may receive pre-abnormality images obtained from an imaging device 301. In some cases, an anatomical mask generation system 302 may generate masks from the pre-abnormality images. Various systems for anatomical segmentation of non-abnormality medical images may be used to produce the anatomical mask based on the pre-abnormality images. In some implementations, processing to generate anatomical masks may occur on processing system logically or physically separate from the SCE 300.

The memory 320 may be used to store: image data 322, descriptors 324, and/or mask data 326 used in synthesis of medical images and abnormality masks. The memory 320 may further store parameters 321, such as machine-learned network states, parameters for abnormality characteristic generation, and/or other synthesis parameters. The memory may further store executable code 329, which may support input data handling, machine-learned network operation and/or other image synthesis functions.

The SCE 300 may also include one or more communication interfaces 312, which may support wireless, e.g. Bluetooth, Bluetooth Low Energy, Wi-Fi, WLAN, cellular (3G, 4G, 5G, LTE/A), and/or wired, ethernet, Gigabit ethernet, optical networking protocols. The SCE 300 may include power management circuitry 334 and one or more input interfaces 328.

The SCE 300 may also include a user interface 318 that may include man-machine interfaces and/or graphical user interfaces (GUI). The GUI may be used to present options for image synthesis generation, such as descriptor input, parameter definitions, machine-learned network training, and/or other operation interactions. The user interface 318, e.g., via the GUI, may generate a presentation of the abnormality masks and/or synthesized images for display on a display device 319 such as a monitor or other display device. Presentation, e.g., as a training interface for interaction, on the display device 319 may facilitate operator review and/or manipulation of the abnormality masks and/or synthesized images. For example, a clinician (or other operator) may view and/or manipulate abnormality masks and/or synthesized images while viewing the user interface 318 via the display device 319. In particular, the description input may be received by the GUI. In some cases, the GUI may also be configured to provide the abnormality spatial mask and the medical image for a medical machine learning system to an operator of the system (e.g., on a display).

The SCE 300 may be implemented as a localized system, in some implementations. In some implementations, the SCE 300 may be implemented as a distributed system. For example, the SCE 300 may be deployed in a cloud computing environment (such as serverless and/or server-centric computing environments). In some cases, the SCE 300 may be implemented (at least in part) on hardware integrated (and/or co-located) with a user terminal.

### Example Implementations

Various example implementations are described below. The example implementations are illustrative of the various general architectures described above. The various specific features of the illustrative example implementations may be readily used in other implementations with or without the various other specific features of the implementation in which they are described.

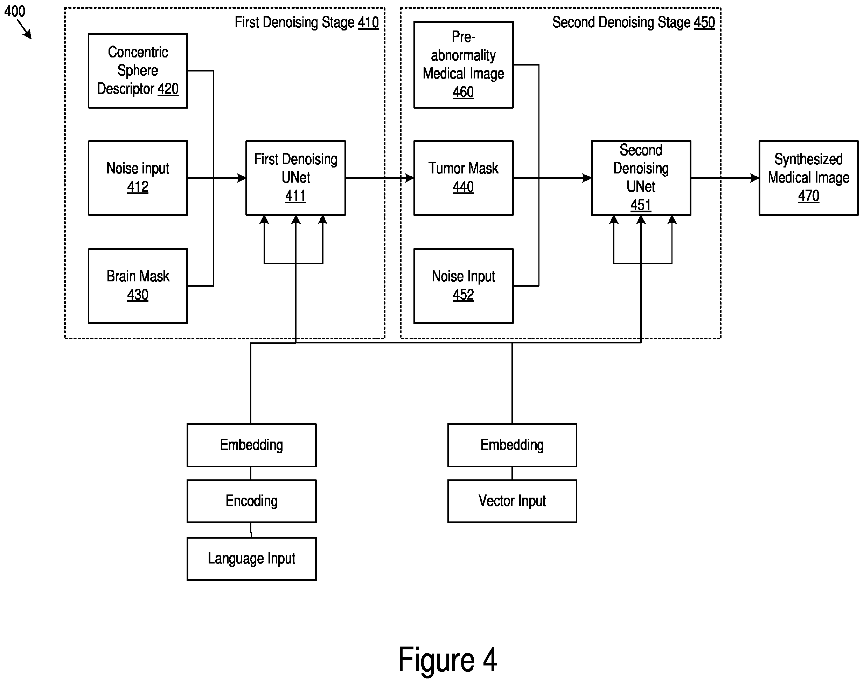

In an illustrative example image synthesis system 400 shown in Figure 4, brain-tumor-type abnormalities are synthesized within medical images of brains. In the illustrative example image synthesis system 400, the two pipeline stages 410, 450 of diffusion model based image synthesis are used. The pipeline uses a set of concentric spheres 420 as the starting point. The illustrative example image synthesis system 400 first synthesizes the multi-label tumor mask 440 from the concentric spheres 512 and then synthesizes the output medical image 470 from the multi-label tumor mask. This example illustrative design enables the model to control the synthetic tumor's location, size and volume ratios of different tumor parts with the configurable parameters of the concentric spheres' location, size and volume ratios, which can synthesize tumors with specified locations, sizes and structures by configuring these parameters.

In the first stage 410, the tumor mask 440 is synthesized from the Gaussian white noise 412 image by iterative denoising operations of the diffusion model 411, where T is the number of iterations. The illustrative example denoising model uses a UNet-like deep convolutional neural network, and shallow N-block unmasked transformers with alternating layers of (i) self-attention, (ii) a position-wise MLP and (iii) a cross-attention layer were added to the lower resolution levels of the UNet. The tumor mask 440 (e.g., which corresponds to the abnormality spatial mask) contains segmentation masks of different parts of tumors with different labels, such as the necrotic and non-enhancing tumor core, the peritumoral edema, the GD-enhancing tumor. The synthesis is conditioned on the anatomical mask, e.g. the brain mask, and a set of concentric spheres 420 by concatenating them with the Gaussian white noise 412 image along the channel dimension. The concentric spheres 420 are used to guide the synthetic tumor mask's location, size, and volume ratios of different tumor portions. The number of concentric spheres used is equal to the number of labels in the tumor mask. The concentric spheres 420 within the descriptor are positioned at the center of mass of the lesion with the volume of each intensity category matching the volume of each label in the tumor mask. The brain mask is used to guide the overall shape and structure of the tumor mask through the tumor mask's relative location in the anatomy, since the tumor's shape and structure have dependence on the tumor's location in the anatomy. The anatomy mask (brain mask 430) can be a single-label mask with the overall mask of the main anatomy the tumor locates in or can be a multi-label mask with both the overall mask of the main anatomy and other masks of the anatomical structures (such as anatomical boundaries) and organs in the main anatomy. Since the illustrative diffusion model is convolutional neural network based, which has the inductive bias of locality and translation equivariance, using concentric spheres as the synthesis starting point is able to encode the controllable location, size and volume ratio information spatially, which is a direct way of encoding parameter for image synthesis.

Additionally or alternatively, indirect vector encoding at a comparatively low dimension may be implemented using conditioning through cross-attention (e.g., input at multiple layers and locations within the neural networks, as discussed below). During inference runtime, the location of the concentric spheres' center can be sampled based on the tumor occurrence probability map relative to the anatomy mask. In this way, the illustrative example system may prevent synthesis of tumors in unrealistic anatomical locations, e.g. brain metastasis in brain ventricles.

In the second stage 450, the denoising model uses a similar diffusion model UNet-like deep convolutional neural network 451 to synthesize the synthetic medical image 470 with a synthetic tumor. The synthesis is conditioned on the tumor mask 440 and the original pre-abnormality image 460, which are concatenated to the Gaussian white noise 452 image along the channel dimension. The original input background medical image 460 guides the synthesis of surrounding tissues around the synthetic tumor and the background image. The guiding from the input background allows the model to generate mass effect around large synthetic tumors. The tumor mask guides the structure of the synthetic tumor and may be used as the ground truth segmentation of the tumor in downstream ML training for tumor segmentation or detection tasks. In some cases, this synthesis step may be conditioned on more than one pre-abnormality image along the channel dimension. They can be of the same subject using different contrasts, the same subject at different longitudinal time points, or other image information from the subject. In the example system, one of the medical images is selected to be the synthetic image's target background. The selected background image 460 may be used in the loss function of the diffusion model.

Mass effect and tissue deformation in the surrounding region of the tumor may be synthesized using abnormality mask segments. For example, outer spheres of the concentric sphere descriptor may designate the volume of regions of non-tumor tissue that are affected by the presence of the tumor. Alternatively, a bounding box or mask applied at the second stage may be used to designate the extent of the effect of the tumor. For example, a bounding box or mask larger than the tumor mask may be defined. The region in the bounding box or mask but outside the tumor mask is the affected region proximate to the tumor, where mass effect and tissue deformation may be present. The region outside the bounding box or mask may be the same as the original pre-abnormality image. In the second stage of the pipeline, after each denoising step the pre-abnormality image can be used to replace the region outside the bounding box or mask in the synthesized image. After replacement, the synthesized image may be used as the input for the next denoising step. Because the background replacement is done after each denoising step, the output synthetic medical image may have a smooth transition across the border formed by the bounding box or mask. In other words, position of the bounding box or mask will not necessarily be apparent, e.g., outside of estimating the position of the box or mask by analyzing where the pre-abnormality image and synthetic image differ.

Additionally or alternatively, denoising stage may use language-based descriptors as input, e.g. clinical reports, electronic health records (EHRs), Digital Imaging and Communication in Medic (DICOM) tags, sequence or protocol parameters or tumor description, and/or other language-based descriptors. The encoded text in the text information may be transformed into a text embedding sequence (e.g., a descriptor vector) using a pretrained language model. The text embedding sequence may applied to the UNet using a transformer (e.g., because the text embedding sequence does not contain image information that can be natively input to the UNet). In some implementations, a multi-head cross-attention application of the text embedding sequence may be implemented by the transformers to apply the text embedding sequence at lower resolution levels of the UNet. Accordingly, the description of the tumor within in the text information may be used to guide the synthetic tumor's appearance. Alternatively, a tumor characteristic vector can be encoded via vector embedding (e.g. rather than using transformation via a language model), and then used to guide the synthetic tumor's appearance. The vector may be applied to the UNet in the same way as the text embedding sequence. The entries of the tumor characteristic vector may represent a characteristic of the tumor, e.g. the level of heterogeneity of the tumor appearance, the contrast of the tumor, the type and subtype of the tumor, the TNM stage of the tumor, whether the tumor is post-operational, an International Classification of Diseases 10^{th} Revision (ICD-10) code, a DICOM tag, sequence or protocol parameters, or other characteristics description. Furthermore, a vector may include a blend of language model extracted characteristics and direct vector entry input.

Optionally, to reduce the computational workload at both training and inference time, the denoising pipeline may be implemented in a down-sampled to a lower resolution and an additional super-resolution step can be applied to the final result to recover the target resolution. Down-sampling followed by upsampling may allow the repeated and computation intensive denoising operations to be performed at a lower resolutions (e.g., which scales down the number of inferences needed by the UNet to produce an output image). Upscaling / super-resolution may add computations to the image synthesis. However, the cumulative reduction in computations across the iterations of the denoising may overcome the additional computation of performing the super-resolution operation. Various image upscaling techniques may be used including a diffusion model upscaling technique. Accordingly, a similar computational environment (to that of the denoising stages) may be used for the super-resolution operation.

In some implementations, the computational workload may be reduced by training an autoencoder to encode all the images, masks and concentric spheres from the original image space into a latent space (e.g., an embedding space similar to the format of the vector descriptors). Then, the image synthesis stages may be implemented within in the latent space. After generating the result, the result will be decoded to the original image space with the decoder in the pretrained auto-encoder.

During inference operation of the denoising stages, classifier-free guidance may be added to further improve the synthetic image quality.

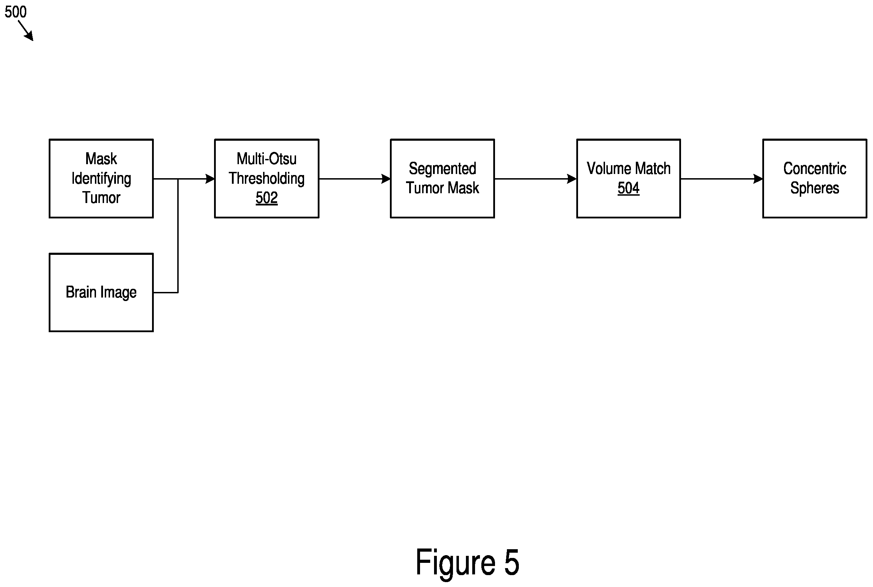

In some implementations, to generate the descriptors used for training the denoising stages, a multi-Otsu thresholding system was used to classify all the voxels within a lesion into multiple intensity categories to generate a quantized intensity map. Then the three concentric spheres were generated at the mass center of the lesion with the volume of each category matching the volume of each intensity group in the quantized intensity map. Figure 5 shows illustrative example training descriptor generation logic 500. In the illustrative example training descriptor generation logic 500, multi-Otsu thresholding classification is performed (502). Then, the volume of segment from the multi-Otsu thresholding is used to generate a corresponding spherical shell (504). For illustrative example purposes, various additional examples of techniques and architectures for generation of masks through thresholding, are described in, U.S. Patent Application No. 18/161,186, filed January 30, 2023, and titled SYNTHESIS OF MEDICAL IMAGES OF BRAIN TUMORS USING 3D-2D GANS, which is incorporated by reference herein in its entirety.

The methods, devices, processing, and logic described in the various sections above may be implemented in many different ways and in many different combinations of hardware and software. For example, all or parts of the implementations may be circuitry that includes an instruction processor, such as a Central Processing Unit (CPU), microcontroller, or a microprocessor; an Application Specific Integrated Circuit (ASIC), Programmable Logic Device (PLD), or Field Programmable Gate Array (FPGA); or circuitry that includes discrete logic or other circuit components, including analog circuit components, digital circuit components or both; or any combination thereof. The circuitry may include discrete interconnected hardware components and/or may be combined on a single integrated circuit die, distributed among multiple integrated circuit dies, or implemented in a Multiple Chip Module (MCM) of multiple integrated circuit dies in a common package, as examples.

The circuitry may further include or access instructions for execution by the circuitry. The instructions may be embodied as a signal and/or data stream and/or may be stored in a tangible storage medium that is other than a transitory signal, such as a flash memory, a Random Access Memory (RAM), a Read Only Memory (ROM), an Erasable Programmable Read Only Memory (EPROM); or on a magnetic or optical disc, such as a Compact Disc Read Only Memory (CDROM), Hard Disk Drive (HDD), or other magnetic or optical disk; or in or on another machine-readable medium. A product, such as a computer program product, may particularly include a storage medium and instructions stored in or on the medium, and the instructions when executed by the circuitry in a device may cause the device to implement any of the processing described above or illustrated in the drawings.

The implementations may be distributed as circuitry, e.g., hardware, and/or a combination of hardware and software among multiple system components, such as among multiple processors and memories, optionally including multiple distributed processing systems. Parameters, databases, and other data structures may be separately stored and managed, may be incorporated into a single memory or database, may be logically and physically organized in many different ways, and may be implemented in many different ways, including as data structures such as linked lists, hash tables, arrays, records, objects, or implicit storage mechanisms. Programs may be parts (e.g., subroutines) of a single program, separate programs, distributed across several memories and processors, or implemented in many different ways, such as in a library, such as a shared library (e.g., a Dynamic Link Library (DLL)). The DLL, for example, may store instructions that perform any of the processing described above or illustrated in the drawings, when executed by the circuitry.

A further aspect of the invention relates to a computer program product comprising instructions which, when the program is executed by a computer, cause the computer to carry out the steps of any method as described elsewhere herein.

Various implementations have been specifically described. However, many other implementations are also possible.

Table 1 includes various examples.

| Table 1: Examples |
|---|
| 1. A system for synthesizing a medical image of a synthesized medical abnormality, the system including: |
| synthesis circuitry configured to: |
| obtain a descriptor input for the synthesized medical abnormality, the descriptor input detailing a selected characteristic for the synthesized medical abnormality; |
| denoise, using a first diffusion model machine-learned network, a first noise input to generate an abnormality spatial mask within a defined multidimensional space; and |
| obtain a pre-abnormality image mapped into the defined multidimensional space; |
| denoise, using a second diffusion model machine-learned network, a second noise input to generate the medical image with the synthesized medical abnormality positioned in accord with abnormality spatial mask; and |
| machine learning control circuitry configured to provide the abnormality spatial mask and the medical image as a training tuple for a medical machine learning system, where: |
| optionally, the system is in accord with the system or method of any other example in this table. |
| 2. The system of example 1 or any other example in this table, where the descriptor input includes a descriptor in a predefined format associated with the first diffusion model machine-learned network. |
| 3. The system of example 2 or any other example in this table, where the predefined format includes: |
| one or more concentric spheres positioned within the defined multidimensional space; and/or |
| a vector indicating one or medical classifications of the synthesized medical abnormality. |
| 4. The system of example 1 or any other example in this table, where the descriptor input includes one or more spheres positioned within the defined multidimensional space to indicate one or more selected volume characteristics and/or a center-of-mass of the synthesized medical abnormality. |
| 5. The system of example 1 or any other example in this table, where: |
| the descriptor input includes a vector descriptor indicating one or medical classifications of the synthesized medical abnormality; and obtaining the descriptor input includes applying a large language model to clinical description of a model medical abnormality to generate the vector descriptor. |
| 6. The system of example 1 or any other example in this table, where the first diffusion model machine-learned network is further configured to denoise the first noise input based on an anatomical mask positioned within the defined multidimensional space, the anatomical mask generated based on the pre-abnormality image. |
| 7. The system of example 6 or any other example in this table, where: |
| the anatomical mask includes a brain mask; and |
| the synthesized medical abnormality includes a brain tumor. |
| 8. The system of example 6 or any other example in this table, where: |
| the anatomical mask includes one or more anatomical boundaries; and the first diffusion model machine-learned network is further configured to denoise the first noise input based on an anatomical mask by positioning and/or shaping the abnormality spatial mask to disallow boundary straddling. |
| 9. The system of example 1 or any other example in this table, where: |
| the first diffusion model machine-learned network is further configured to denoise the first noise input iteratively using multiple denoising iterations; and |
| the second diffusion model machine-learned network is further configured to denoise the second noise input iteratively using multiple denoising iterations. |
| 10. The system of example 1 or any other example in this table, where the first diffusion model machine-learned network and/or second diffusion model machine-learned network include diffusion model machine-learned networks trained using image set generated using a ground truth image with increasing levels of noise added. |
| 11. The system of example 1 or any other example in this table, where the synthesized medical abnormality includes a tumor, a lung nodule, and/or a lesion. |
| 12. The system of example 1 or any other example in this table, where the pre-abnormality image includes a magnetic resonance imaging (MRI) image and/or a computerized tomography (CT) image. |
| 13. The system of example 1, where the defined multidimensional space includes a two-dimensional space or a three-dimensional space. |
| 14. A multiple-stage denoising method for synthesizing a medical image of a synthesized medical abnormality, the method including: |
| denoising, using a first diffusion model machine-learned network at a first denoising stage, a first noise input to obtain an abnormality spatial mask within a defined multidimensional space; |
| after obtaining the abnormality spatial mask, denoising, using a pre-abnormality image and a second diffusion model machine-learned network at a second denoising stage, a second noise input to obtain the medical image of the synthesized medical abnormality, the medical image consistent with pre-abnormality image modified to include the synthesized medical abnormality inserted in accord with the abnormality spatial mask; and |
| providing the medical image to a training interface for training interaction, where: |
| optionally, the method is in accord with the system or method of any other example in this table. |
| 15. The multiple-stage denoising method of example 14 or any other example in this table, where denoising the second noise input to obtain the medical image include obtaining an image to supplement a training set of medical images with deficient occupancy for medical images with a medical abnormality with at least a selected characteristic present in the synthesized medical abnormality. |
| 16. The multiple-stage denoising method of example 15 or any other example in this table, where the deficient occupancy of the training set includes: |
| a deviation from a medically established relative probability for occurrences of the medical abnormality with at least the selected characteristic; |
| an absence of medical images with the medical abnormality with at least the selected characteristic; and |
| a below threshold amount of total images within the training set. |
| 17. The multiple-stage denoising method of example 14 or any other example in this table, where denoising the first noise input to obtain the abnormality spatial mask includes denoising the first noise input to obtain the abnormality spatial mask within an anatomical mask positioned within the defined multidimensional space. |
| 18. The multiple-stage denoising method of example 17 or any other example in this table, where: |
| the anatomical mask includes one or more anatomical boundaries; and at a time that the abnormality has a center-of-mass near the one or more anatomical boundaries: |
| denoising the first noise input includes shaping the abnormality spatial mask to disallow boundary straddling; and/or |
| denoising the second noise input includes deforming a portion of the medical image relative to the pre-abnormality image to shift one or more portions of the medical image associated with the anatomical boundary to disallow boundary straddling. |
| 19. The multiple-stage denoising method of example 17 or any other example in this table, where: |
| the anatomical mask includes a brain mask; and |
| the abnormality includes a brain tumor. |
| 20. A denoising method for synthesizing a medical image of a synthesized medical abnormality with a selected characteristic, the method including: |
| obtaining a descriptor of at least the selected characteristic of the synthesized medical abnormality in a predefined format associated with a first diffusion model machine-learned network; |
| providing the descriptor and a first noise input to the first diffusion model machine-learned network; |
| denoising, via the first diffusion model machine-learned network, the first noise input to obtain an abnormality spatial mask that spatially defines the synthesized medical abnormality with the selected characteristic; |
| after obtaining the abnormality spatial mask, denoising, using a pre-abnormality image and a second diffusion model machine-learned network, second noise input to obtain the medical image of the synthesized medical abnormality, the medical image consistent with pre-abnormality image modified to include the synthesized medical abnormality inserted in accord with the abnormality spatial mask; and |
| providing the medical image to a training interface for training interaction, where: |
| optionally, the method is in accord with the system or method of any other example in this table. |
| 21. A system including circuitry configured to implement any feature or any combination of features described in this table or disclosure. |
| 22. A method including implementing any feature or any combination of features described in this table or disclosure. |
| 23. A method including installing the system of any example in this table. |
| 24. A product including: |
| machine-readable media; and |
| instructions stored on the machine-readable media, the instructions configured to cause a processor to perform (at least in part) the method of any example in this table, where: |
| optionally, the machine-readable media is non-transitory; |
| optionally, the machine-readable media is other than a transitory signal; and |
| optionally, the instructions are executable. |

Embodiments described with respect to a method equally apply to a respective apparatus and/or a system which may be configured to execute respective method steps and vice versa. In some embodiments, the system described herein may comprise means for executing the method described herein. More specifically, in some embodiments, the system of examples 1-13 and 21 may be configured to (e.g., may comprise means to) execute the steps of the method according to any one of the examples 14 - 20 or examples 22 or 23 of the above table 1. It is understood that the system as claimed, in embodiments, is configured to carry out the methods steps according to the method as claimed.

Headings and/or subheadings used herein are intended only to aid the reader with understanding described implementations. The invention is defined by the claims.

## Claims

1. A system (100) for synthesizing a medical image (170) of a synthesized medical abnormality, the system including:
synthesis circuitry (314) configured to:
obtain (212) a descriptor input (120) for the synthesized medical abnormality, the descriptor input (120) detailing a selected characteristic for the synthesized medical abnormality;
denoise (210), using a first diffusion model machine-learned network (110), a first noise input (112) to generate an abnormality spatial mask (140) within a defined multidimensional space; and
obtain a pre-abnormality image (160) mapped into the defined multidimensional space;
denoise (220), using a second diffusion model machine-learned network (150), a second noise input (152) to generate the medical image with the synthesized medical abnormality positioned in accord with the abnormality spatial mask (140); and
machine learning control circuitry (318) configured to provide (230) the abnormality spatial mask and the medical image for a medical machine learning system.

2. The system of claim 1, where the descriptor input (120) includes a descriptor in a predefined format associated with the first diffusion model machine-learned network (110).

3. The system of claim 2, where the predefined format includes:
one or more concentric spheres positioned within the defined multidimensional space; and/or
a vector indicating one or medical classifications of the synthesized medical abnormality.

4. The system of one of the claims 1-3, where the descriptor input (120) includes one or more spheres positioned within the defined multidimensional space to indicate one or more selected volume characteristics and/or a center-of-mass of the synthesized medical abnormality.

5. The system of one of the claims 1-4, where:
the descriptor input (120) includes a vector descriptor indicating one or medical classifications of the synthesized medical abnormality; and
obtaining the descriptor input includes applying a large language model to clinical description of a model medical abnormality to generate the vector descriptor.

6. The system of one of the claims 1-5, where the first diffusion model machine-learned network (110) is further configured to denoise (210) the first noise input based on an anatomical mask (130) positioned within the defined multidimensional space, the anatomical mask generated based on the pre-abnormality image.

7. The system of claim 6, where:
the anatomical mask (130) includes a brain mask (430); and
the synthesized medical abnormality includes a brain tumor; and/or where: the anatomical mask (130) includes one or more anatomical boundaries; and
the first diffusion model machine-learned network (110) is further configured to denoise the first noise input (112) based on an anatomical mask by positioning and/or shaping the abnormality spatial mask to disallow boundary straddling.

8. The system of one of the claims 1-7, where:
the first diffusion model machine-learned network (110) is further configured to denoise the first noise input (112) iteratively using multiple denoising iterations; and
the second diffusion model machine-learned network (150) is further configured to denoise the second noise input (152) iteratively using multiple denoising iterations; and/or where the first diffusion model machine-learned network (110) and/or second diffusion model machine-learned network include diffusion (150) model machine-learned networks trained using image set generated using a ground truth image with increasing levels of noise added.

9. The system of one of the claims 1-8, where the synthesized medical abnormality includes a tumor, a lung nodule, and/or a lesion; and/or
where the pre-abnormality image (160) includes a magnetic resonance imaging, MRI, image and/or a computerized tomography, CT, image; and/or
where the defined multidimensional space includes a two-dimensional space or a three-dimensional space.

10. A multiple-stage denoising method (200) for synthesizing a medical image (170) of a synthesized medical abnormality, the method including:
denoising (210), using a first diffusion model machine-learned network at a first denoising stage (110), a first noise input (112) to obtain an abnormality spatial mask (140) within a defined multidimensional space;
after obtaining the abnormality spatial mask (140), denoising (220), using a pre-abnormality image (160) and a second diffusion model machine-learned network at a second denoising stage (150), a second noise input to obtain the medical image of the synthesized medical abnormality, the medical image (170) consistent with pre-abnormality image (160) modified to include the synthesized medical abnormality inserted in accord with the abnormality spatial mask (140); and
providing (230) the medical image to a training interface (318) for training interaction.

11. The multiple-stage denoising method of claim 10, where denoising (220) the second noise input (152) to obtain the medical image include obtaining an image to supplement a training set of medical images with deficient occupancy for medical images with a medical abnormality with at least a selected characteristic present in the synthesized medical abnormality.

12. The multiple-stage denoising method of claim 11, where the deficient occupancy of the training set includes:
a deviation from a medically established relative probability for occurrences of the medical abnormality with at least the selected characteristic;
an absence of medical images with the medical abnormality with at least the selected characteristic; and
a below threshold amount of total images within the training set.

13. The multiple-stage denoising method of one of the claims 10 - 12, where denoising (210) the first noise input (152) to obtain the abnormality spatial mask (140) includes denoising (210) the first noise input (152) to obtain the abnormality spatial mask (140) within an anatomical mask (130) positioned within the defined multidimensional space.

14. The multiple-stage denoising method of claim 13, where:
the anatomical mask (130) includes one or more anatomical boundaries; and
at a time that the abnormality has a center-of-mass near the one or more anatomical boundaries:
denoising (210) the first noise input (112) includes shaping the abnormality spatial mask (130) to disallow boundary straddling; and/or
denoising (220) the second noise input (152) includes deforming a portion of the medical image (170) relative to the pre-abnormality image (160) to shift one or more portions of the medical image (170) associated with the anatomical boundary to disallow boundary straddling; and/or where:
the anatomical mask (130) includes a brain mask (430); and
the abnormality includes a brain tumor.

15. The multiple-stage denoising method of one of the claims 10 - 14, wherein the synthesized medical abnormality further comprises a selected characteristic and the method further comprising:
obtaining a descriptor (120) of at least the selected characteristic of the synthesized medical abnormality in a predefined format associated with a first diffusion model machine-learned network (110);
providing the descriptor (120) and a first noise input to the first diffusion model machine-learned network (110);
wherein the abnormality spatial mask spatially defines the synthesized medical abnormality with the selected characteristic.
